(19) **Europäisches Patentamt**

**European Patent Office**

**Office européen des brevets**

(11) **EP 1 561 949 A1**

(12) **EUROPEAN PATENT APPLICATION**
published in accordance with Art. 158(3) EPC

(43) Date of publication:
**10.08.2005 Bulletin 2005/32**

(51) Int Cl.⁷: **F04B 49/06**, A61M 5/14

(21) Application number: **03811071.4**

(86) International application number:
**PCT/JP2003/014110**

(22) Date of filing: **05.11.2003**

(87) International publication number:
**WO 2004/044426 (27.05.2004 Gazette 2004/22)**

(84) Designated Contracting States:
**AT BE BG CH CY CZ DE DK EE ES FI FR GB GR
HU IE IT LI LU MC NL PT RO SE SI SK TR**

(30) Priority: **11.11.2002 JP 2002327445**

(71) Applicant: **KABUSHIKI KAISHA TOP
Tokyo 120-0035 (JP)**

(72) Inventor: **KOBAYASHI, Susumu,
c/o KABUSHIKI KAISHA TOP
Tokyo 120-0035 (JP)**

(74) Representative: **Henkel, Feiler & Hänzel
Möhlstrasse 37
81675 München (DE)**

(54) **SYRINGE PUMP**

(57) Travel amount detecting means 10 for detecting the travel amount of a plunger 4b, distal end detecting means 11 for detecting the fact that the plunger 4b has moved to the distal end of a syringe 4, distance measuring means for measuring the travel distance of the plunger 4b, input means 13 capable of inputting the capacity of the syringe 4, and storage means 14 capable of storing the measured travel distance of the plunger 4b and the input capacity of the syringe 4 are provided.

The control means 16 selects the travel distance of the plunger 4b corresponding to the input capacity from the storage means 14 and controls the injection of a chemical solution in the syringe 4 accomplished via plunger pressing means based on the travel distance of plunger and the capacity of syringe. Thereby, a syringe pump can be provided which can be used for a syringe that is sold newly or a syringe with a dimension changed by design change or remodeling.

FIG. 1

EP 1 561 949 A1

**Description**

TECHNICAL FIELD

The present invention relates to a syringe pump that injects a chemical solution filled in a syringe, continuously at a preset flow rate.

BACKGROUND ART

Conventionally, a syringe pump having storage means, control means, and plunger pressing means has been known (for example, see Japanese Patent Laid-Open No. 2000-316971). Usually, in the storage means of the above-described syringe pump are stored predetermined data including the capacity of syringe commercially available at the time when the syringe pump is manufactured, and the travel distance of a plunger from the maximum scale position of syringe to the distal end position (minimum scale position) of syringe.

Such a syringe is operated, for example, as described below. First, the operator inputs the capacity of syringe from an input section. The input capacity is used to select the corresponding syringe data from the storage means. The plunger inserted in the syringe is pressed and moved in the axial direction by using the plunger pressing means to inject a chemical solution within the syringe while the plunger pressing means is controlled by the control means based on the selected syringe data so that the chemical solution is injected from the syringe at a preset flow rate.

However, in the conventional syringe pump, the syringe data stored in the storage means of syringe pump is limited to syringes commercially available at the time when the syringe pump is manufactured. Therefore, the conventional syringe pump has a disadvantage of being incapable of being used for a syringe that is sold newly after that time or a syringe with a dimension changed by design change or remodeling.

The present invention has been made in view of the above background, and accordingly an object thereof is to provide a syringe pump which can be used for a syringe that is sold newly or a syringe with a dimension changed by design change or remodeling.

DISCLOSURE OF THE INVENTION

The present invention provides a syringe pump comprising a holding portion for holding a syringe provided with a scale in its peripheral wall for checking the amount of a chemical solution filled therein, plunger pressing means for pressing and moving a plunger inserted in the syringe held by the holding portion, in the axial direction to inject the chemical solution in the syringe, control means for controlling the plunger pressing means so that the plunger pressing means presses the plunger continuously at a predetermined speed, travel amount detecting means for detecting the travel amount of the plunger, distal end detecting means for detecting the fact that the plunger has moved to the distal end of syringe, distance measuring means for measuring the travel distance of the plunger based on the travel amount detecting means and the distal end detecting means, input means capable of inputting the capacity of syringe, and storage means capable of storing the travel distance of plunger measured by the distance measuring means and the capacity of syringe input by the input means, and the control means comprising means for controlling the injection of chemical solution in the syringe accomplished via the plunger pressing means based on the travel distance of the plunger and the capacity of the syringe.

According to the above-described invention, the syringe is set on the holding portion, and the capacity of the syringe is input. Then, the distal end of the plunger is set at the maximum scale position of the syringe.

Next, the plunger is pressed in the axial direction by the plunger pressing means. When the plunger moves to the distal end of the syringe, the distal end of the syringe is detected by the distal end detecting means. Based on the outputs of the travel amount detecting means and the distal end detecting means, the travel distance of the plunger is measured by the distance measuring means.

Next, the input capacity of syringe and the measured travel distance of plunger are stored in the storage means. If at least the capacity of syringe and the travel distance of plunger are found, the amount of the chemical solution injected from the syringe can be judged by comparing the whole travel distance of plunger with the current travel distance thereof. Therefore, the chemical solution can be injected continuously at a preset flow rate from the distal end of the syringe based on the input capacity of syringe and the measured travel distance of plunger.

When the syringe pump in accordance with the present invention is used, the syringe is held by the holding portion, and the capacity of syringe is input by the input means. Then, the control means identifies the travel distance of the plunger corresponding to the capacity of the syringe from the storage means. Then, based on the capacity of the syringe and the travel distance of the plunger, the control means controls the plunger pressing means, by which the chemical solution is injected continuously at a preset flow rate from the distal end of the syringe.

Thereby, the syringe pump in accordance with the present invention can be used even for a syringe that is sold newly or a syringe with a dimension changed by design change or remodeling.

Also, since the syringe pump in accordance with the present invention is configured so that the data on the syringe can be stored freely by the operator, there is a fear that mistaken data on the syringe is stored by the operator. Specifically, an error of travel distance of the plunger occurs, for example, because the plunger is not set at the maximum scale position when the syringe is stored, or the input of the capacity of syringe is mistaken.

For this reason, the syringe pump in accordance with the present invention preferably comprises outside diameter detecting means for detecting the outside diameter of the syringe held by the holding portion, capacity calculating means for calculating the capacity of the syringe based on the outside diameter of syringe detected by the outside diameter detecting means and the travel distance of plunger measured by the distance measuring means, difference calculating means for calculating a difference between the capacity of syringe calculated by the capacity calculating means and the capacity of syringe input by the input means, and registration means which accepts the storage in the storage means if the difference in capacity of syringe calculated by the difference calculating means is within a predetermined range.

The outside diameter detecting means measures the outside diameter of the syringe held by the holding portion. The capacity calculating means calculates the capacity of the syringe based on the outside diameter of syringe detected by the outside diameter detecting means and the travel distance of plunger measured by the distance measuring means. At this time, in order to calculate the capacity of syringe, it is necessary to calculate the inside diameter of syringe by subtracting the wall thickness of syringe from the outside diameter of syringe. The wall thickness of syringe is determined in advance because it is almost constant for any syringe.

The difference calculating means calculates a difference in capacity by comparing the capacity of syringe calculated by the capacity calculating means with the capacity of syringe input by the input means. The registration means accepts the storage of the outside diameter of syringe measured by the outside diameter detecting means, the travel distance of plunger measured by the distance measuring means, and the capacity of syringe input by the input means in the storage means if the difference in capacity of syringe calculated by the difference calculating means is within a predetermined range. Thereby, a fear that the operator stores the mistaken data on the syringe in the storage means can be eliminated.

Also, the storage means preferably comprises an initial syringe data storage section which stores in advance predetermined data including the outside diameters of predetermined syringes of a plurality of kinds, and at least the capacity of syringe and the travel distance of plunger, which correspond to the syringe of each outside diameter.

Thereby, for the syringe that has already been sold, the data can be stored in the initial syringe data storage section on the maker side. Therefore, work for storing the already sold syringe in the storage means by the operator himself can be omitted.

BRIEF DESCRIPTION OF THE DRAWINGS

FIG. 1 is an explanatory view showing a basic construction of a syringe pump in accordance with one embodiment of the present invention;
FIG. 2 is an explanatory sectional view of outside diameter detecting means in accordance with one embodiment of the present invention, viewed from the syringe distal end side;
FIG. 3 is a block diagram showing a configuration of storage means and control means in accordance with one embodiment of the present invention;
FIG. 4 is a flowchart for illustrating a process for storing a syringe into a syringe pump in accordance with one embodiment of the present invention; and
FIG. 5 is a flowchart for illustrating an operation for injecting a chemical solution from a syringe by using a syringe pump in accordance with one embodiment of the present invention.

BEST MODE FOR CARRYING OUT THE INVENTION

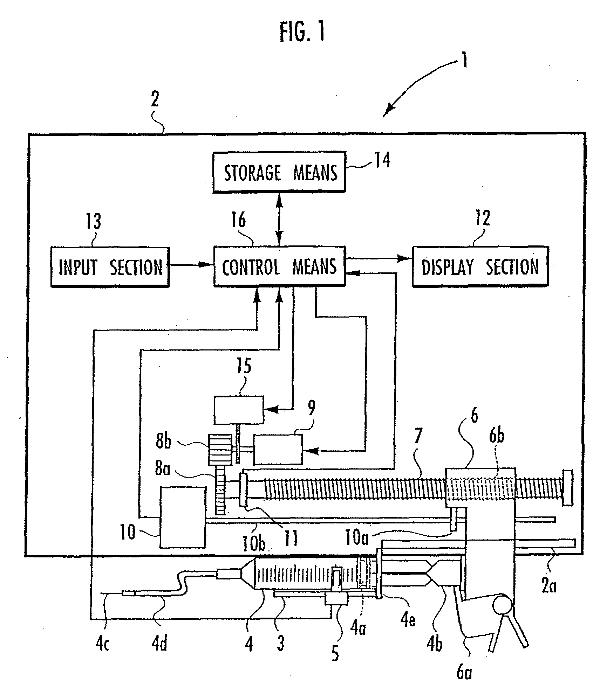

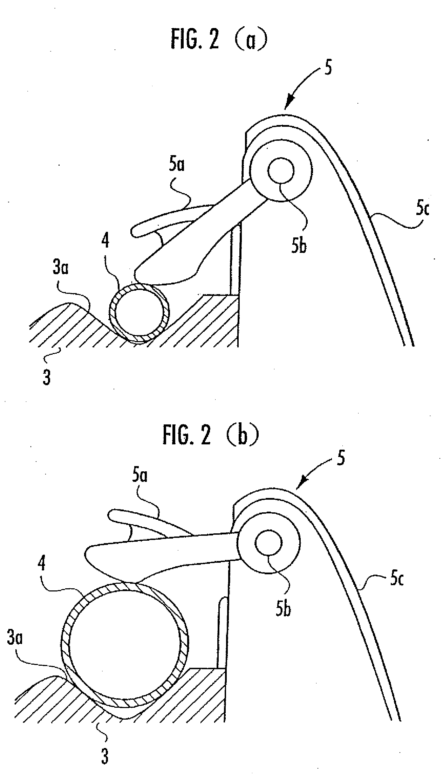

As shown in FIG. 1, a syringe pump 1 in accordance with an embodiment of the present invention includes a syringe pump body 2 and a syringe holding portion 3 provided on the side surface of the syringe pump body 2. As shown in FIG. 2, the syringe holding portion 3 has a concave 3a having a substantially semicircular shape in cross section with the concave 3a parallel with the body side surface so that a syringe 4 can be put on the concave 3a. As shown in FIG. 1, a plunger 4b having a gasket 4a at its distal end is inserted in the syringe 4. To the distal end of the syringe 4, a tube 4d having a piercing instrument 4c at its distal end is connected. Also, the syringe 4 has a flange 4e at the proximal end of outer cylinder thereof.

In an outside edge portion of the syringe holding portion 3, a syringe size sensor 5 is provided as outside diameter detecting means. As shown in FIGS. 2(a) and 2(b), the syringe size sensor 5 includes an arm portion 5a, a shaft 5b, and a support 5c. The proximal end of the arm portion 5a is rotatably connected to the support 5c by the shaft 5b so as to intersect perpendicularly to the axis of the syringe 4. The syringe 4 is held between the arm portion 5a and the syringe holding portion 3. The syringe size sensor 5 detects the outside diameter of the syringe 4 by means of the rotation angle of the shaft 5b.

Also, as shown in FIG. 1, a rectangularly shaped opening 2a is provided in the body side surface so as to be parallel with the axial direction of the syringe 4. In the opening 2a, a plunger presser 6 having a plunger clamp 6a at its distal end is inserted from the proximal end thereof. The plunger clamp 6a can be engaged with the rear end of the plunger 4b.

In the proximal end portion of the plunger presser 6, a screw hole 6b is provided so as to be parallel with the opening 2a. A ball screw member 7 is screwed in the screw hole 6b. The ball screw member 7 and the opening 2a are provided so as to be parallel with each other. Also, the opening 2a guides the plunger presser 6 so that the plunger presser 6 is moved in the axial direction of the ball screw member 7. Therefore, the rotation of the plunger presser 6 around the ball screw member 7 is inhibited. At the distal end of the ball screw member 7 is fitted a gear 8a. With the gear 8a, a gear 8b installed to a motor 9 meshes. The ball screw member 7 is rotated by the motor 9 via the gears 8a and 8b. The rotational motion of the motor 9 is converted to a linear motion of the plunger presser 6 moving in the axial direction of the ball screw member 7 by the screw hole 6b via the ball screw member 7.

In this embodiment, the opening 2a, the plunger presser 6, the ball screw member 7, the gears 8a and 8b, and the motor 9 constitute plunger pressing means.

Travel amount detecting means 10 of this embodiment includes a contact 10a and a linear potentiometer 10b. The contact 10a is projectingly provided in the proximal end portion of the plunger presser 6. Also, the linear potentiometer 10b is provided in the syringe pump body 2 so as to be parallel with the axis of the ball screw member 7 and to be in contact with the contact 10a. The plunger 4b is pressed by the plunger presser 6, and the contact 10a provided on the plunger presser 6 slides on the linear potentiometer 10b. The electrical resistance of the linear potentiometer 10b changes according to the position of the contact 10a, by which the travel amount of the plunger 4b is detected.

In the distal end portion of the ball screw member 7, a load sensor 11 is provided as distal end detecting means. When the gasket 4a moves to the distal end of the syringe 4, the plunger 4b cannot move in the distal end direction of the syringe 4, so that a load is applied to the rotation of the ball screw member 7 caused by the motor 9. The load sensor 11 detects the fact that the gasket 4a has been moved to the distal end of the syringe 4 by this load.

A display section 12 is provided on the syringe pump body 2. The display section 12 displays a setting screen menu, the out side diameter of the syringe 4 held by the syringe holding portion 3, the name of maker, the travel distance of the plunger 4b, the capacity of the syringe 4, and the like. On the syringe pump body 2, an input section 13 is provided as input means. To the input section 13, the capacity of the syringe 4 and the name of maker can be input.

In this embodiment, as shown in FIG . 3, for example, a RAM disc 14a is incorporated in the syringe pump body 2 as storage means 14. The RAM disc 14a can store the outside diameter of the syringe 4, the travel distance of the plunger 4b, the capacity of the syringe 4, and the name of maker of the syringe 4. Also, the RAM disc 14a is provided with a battery so as to maintain the storage even if the power source of the syringe pump 1 is turned off.

Also, the rotational speed of the motor 9 is controlled by a motor encoder 15.

In this embodiment, the syringe pump body 2 contains a ROM 17, a CPU 18, and a RAM 19.

In the ROM 17, there are written a distance calculation program 17a, a capacity calculation program 17b, a difference calculation program 17c, a registration program 17d, and a control program 17e, which are executed by using the CPU 18 and the RAM 19.

The distance calculation program 17a calculates the travel distance of the plunger 4b from the maximum scale position to the syringe distal end position based on the travel amount output from the travel amount detecting means 10 and the load sensor 11 (in this embodiment, the positional information from the maximum scale position to the syringe distal end position).

The capacity calculation program 17b calculates the capacity of the syringe 4 based on the travel distance of the plunger 4b calculated by the distance calculation program 17a and the outside diameter of the syringe 4 measured by the syringe size sensor 5 by using Equation (1) described below. In Equation (1), t is a constant value determined considering the wall thickness of the syringe 4.

$$V = (D - 2 \times t)2 \times \pi \times L/4 \qquad (1)$$

where,

V:    calculated capacity
D:    measured outside diameter
t:     predetermined wall thickness data of syringe
L:     travel distance of plunger

The difference calculation program 17c calculates a difference in capacity by comparing the capacity of syringe 4 input by the input section 13 with the capacity of syringe 4 calculated by the capacity calculation program 17b.

If the difference in capacity calculated by the difference calculation program 17c is within a predetermined range, the registration program 17d accepts the storage of the outside diameter of the syringe 4, the travel distance of the plunger 4b, the capacity of the syringe 4, and the name of maker of the syringe 4 in the RAM disc 14a. If the difference in capacity is out of the predetermined range, the registration program 17d refuses the storage of the outside diameter of the syringe 4, the travel distance of the plunger 4b, the capacity of the syringe 4, and the name of maker of the syringe 4 in the RAM disc 14a.

The control program 17e controls the motor encoder 15 so that a chemical solution is injected continuously from the syringe 4 based on the preset flow rate input by the input section 13.

Also, predetermined data consisting of the outside diameters of a plurality of syringes 4, and the travel distance of the plunger 4b which correspond to each outside diameter, the capacity of the syringe 4, and the name of maker of the syringe 4 are written in advance in the ROM 17 as an initial syringe data storage section 14b. That is to say, in this embodiment, a part of the ROM 17 constitutes a part of the storage means 14.

In this embodiment, the motor encoder 15, the ROM 17, the CPU 18, and the RAM 19 constitute control means 16.

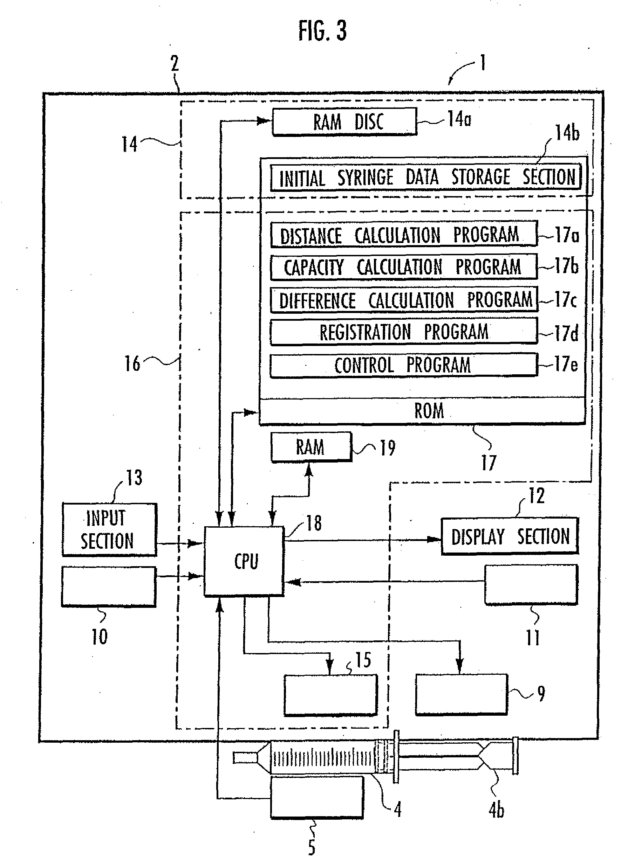

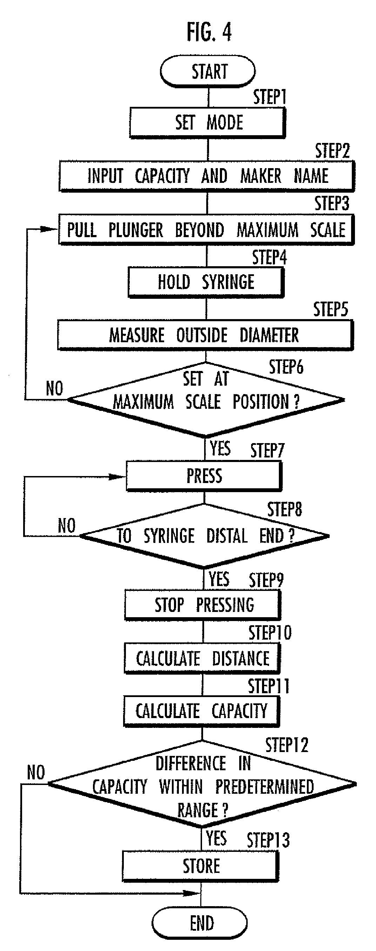

Next, a process for storing the syringe 4 in the syringe pump 1 of this embodiment will be described with reference to FIGS. 1, 3 and 4.

First, the power source for the syringe pump 1 is turned on. Then, the control proceeds to Step 1, where a setting mode is selected. Then, the control proceeds to Step 2, where the capacity described on the scale of the syringe 4 and the name of maker of the syringe 4 are input by the input section 13.

Next, the control proceeds to Step 3, where the gasket 4a is pulled beyond the maximum scale position via the plunger 4b. Then, the control proceeds to Step 4, where the syringe 4 is held by the syringe holding portion 3. Then, the control proceeds to Step 5, where the outside diameter of the syringe 4 is measured by the syringe size sensor 5.

Next, the control proceeds to Step 6, where the distal end of the gasket 4a is set at the maximum scale position of the syringe 4 by operating the input section 13. If the distal end of the plunger 4b is brought too far beyond the maximum scale position by a mistaken operation of the input section 13, the control returns to Step 3. After the distal end of the plunger 4b has been set at the maximum scale position, the control proceeds to Step 7, where the maximum scale position is stored in the RAM 19. Also, the motor 9 rotates the ball screw member 7 via the gears 8a and 8b while being controlled by the motor encoder 15, so that the plunger presser 6 presses the plunger 4b. Next, the control proceeds to Step 8, where the load on the ball screw member 7 is monitored by the load sensor 11 to judge whether or not the gasket 4a has moved to the distal end of the syringe 4.

If the load on the ball screw member 7 is detected by the load sensor 11, the control proceeds to Step 9, where the motor 9 is stopped to stop pressing of the plunger 4b by the plunger presser 6. If the load on the ball screw member 7 is not detected by the load sensor 11, the control returns to Step 7 , and hence the pressing of the plunger 4b is continued.

Next, the control proceeds to Step 10, where the position of the plunger 4b at the time when it is detected by the load sensor 11 that the gasket 4a has moved to the distal end of the syringe 4 is output by the contact 10a and the linear potentiometer 10b. Then, the travel distance of the plunger 4b is calculated by the distance calculation program 17a based on the maximum scale position stored in the RAM 19 and the position output by the load sensor 11. Next, the control proceeds to Step 11, where the capacity of the syringe 4 is calculated by the capacity calculation program 17b based on the outside diameter of the syringe 4 measured by the syringe size sensor 5 and the travel distance of the plunger 4b calculated by the distance calculation program 17a. Then, the control proceeds to Step 12, where the difference calculation program 17c calculates a difference in capacity by comparing the capacity calculated by the capacity calculation program 17b with the capacity input by the input section 13 by the operator in Step 2.

Next, if the difference in capacity calculated by the difference calculation program 17c is within a predetermined range, the registration program 17d accepts the storage of the outside diameter of the syringe 4 measured by the syringe size sensor 5, the capacity of the syringe 4 which corresponds to the outside diameter of the syringe 4 and is input in Step 2, the name of maker of the syringe 4 which is similarly input in Step 2, and the travel distance of the plunger 4b calculated by the distance calculation program 17a in the RAM disc 14a. Inversely, if the difference in capacity is out of the predetermined range, the registration program 17d refuses the storage of the outside diameter of the syringe 4, the capacity of the syringe 4 which corresponds to the outside diameter of the syringe 4, the name of maker of the syringe 4, and the travel distance of the plunger 4b in the RAM disc 14a.

Next, the display section 12 displays the result of whether or not the outside diameter of the syringe 4, the capacity of the syringe 4 which corresponds to the outside diameter of the syringe 4, the name of maker of the syringe 4, and the travel distance of the plunger 4b have been stored in the RAM disc 14a.

Thus, the process for storing the syringe 4 in the syringe pump 1 is completed.

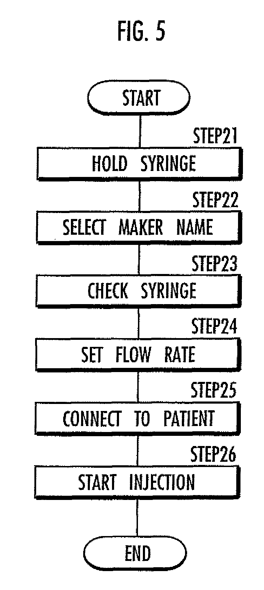

Next, a case where a patient is injected with a chemical solution by using the syringe pump 1 of this embodiment will be described with reference to FIGS. 1 and 5.

First, the power source for the syringe pump 1 is turned on. Then, the control proceeds to Step 21, where the syringe 4 filled with the chemical solution is held by the syringe holding portion 3. Then, the control proceeds to Step 22, where the name of maker of the syringe 4 is selected by the input section 13. Next, the control proceeds to Step 23, where the outside diameter of the syringe 4 is measured by the syringe size sensor 5. The capacity of the syringe 4 corresponding to the measured outside diameter of the syringe 4 and the travel distance of the plunger 4b are displayed on the display section 12. Then, the control proceeds to Step 24, where the flow rate per predetermined time of chemical solution injected from the syringe 4 is set. Next, the control proceeds to Step 25, where the piercing instrument 4c is connected to the patient. Then, the control proceeds to Step 26, where the injection is started by the operation of the input section 13.

According to the syringe pump of this embodiment, a syringe that is sold newly or a syringe with a dimension changed by design change or remodeling can be stored. Also, if the syringe 4 is once stored in the RAM disc 14a, the information stored in the RAM disc 14a can be read next time and subsequently, so that the stored syringe can be used without troublesome setting/adjusting operation. Also, according to the syringe pump of this embodiment, by storing the outside diameter of the syringe 4 that is commercially available at the time when the syringe pump is manufactured and the capacity corresponding to the outside diameter in advance in the ROM 17, the operator' s burden of inputting can be eased.

In this embodiment, the syringe pump 1 using, for example, the RAM disc 14a as the storage means 14 has been explained. However, any storage unit that is writable, such as a magnetic disc, a flash memory, an optical disc, can be used.

Also, in this embodiment, the syringe pump 1 in which the initial syringe data storage section 14b is provided in the ROM 17 has been explained. However, the initial syringe data storage section 14b may be provided at another place, for example, on the RAM disc 14a.

Also, in this embodiment, the syringe pump 1 provided with the contact 10a and the linear potentiometer 10b as the travel amount detecting means has been explained. However, in place of providing the contact 10a and the linear potentiometer 10b, the travel amount of the plunger may be detected by detecting the rotational speed of the motor by using the motor encoder 15. In this case, the distance calculation program calculates the travel distance of the plunger from the rotational speed based on the screw pitch of the ball screw member 7. Also, in place of providing the contact 10a and the linear potentiometer 10b, a step motor may be used instead of the motor 9 to detect the number of pulses, thereby detecting the travel amount of the plunger. In this case, the distance calculation program calculates the travel distance of the plunger by determining the rotational speed of the step motor from the number of pulses.

INDUSTRIAL APPLICABILITY

According to the present invention, a syringe that is sold newly or a syringe with a dimension changed by design change or remodeling can be stored. Therefore, the stored syringe can be used without troublesome setting/adjusting operation, so that in medical treatment such as injection of patient with a chemical, smooth and exact injection of chemical solution can be accomplished.

**Claims**

1. A syringe pump comprising:

   a holding portion for holding a syringe provided with a scale in its peripheral wall for checking the amount of a chemical solution filled therein;
   plunger pressing means for pressing and moving a plunger inserted in the syringe held by said holding portion, in the axial direction to inject the chemical solution in the syringe;
   control means for controlling said plunger pressing means so that said plunger pressing means presses the plunger continuously at a predetermined speed,
   travel amount detecting means for detecting the travel amount of the plunger;
   distal end detecting means for detecting the fact that the plunger has moved to the distal end of syringe;
   distance measuring means for measuring the travel distance of the plunger based on said travel amount detecting means and said distal end detecting means;
   input means capable of inputting the capacity of syringe; and
   storage means capable of storing the travel distance of plunger measured by said distance measuring means and the capacity of syringe input by said input means, and
   said control means comprising means for controlling the injection of chemical solution in the syringe accomplished via said plunger pressing means based on the travel distance of the plunger and the capacity of the syringe.

2. The syringe pump according to claim 1 comprising:

   outside diameter detecting means for detecting the outside diameter of the syringe held by said holding portion;
   capacity calculating means for calculating the capacity of the syringe based on the outside diameter of syringe detected by said outside diameter detecting means and the travel distance of plunger measured by said distance measuring means;
   difference calculating means for calculating a difference between the capacity of syringe calculated by said capacity calculating means and the capacity of syringe input by said input means; and
   registration means which accepts the storage in said storage means if the difference in capacity of syringe calculated by said difference calculating means is within a predetermined range.

3.  The syringe pump according to claim 2, wherein said storage means comprises an initial syringe data storage section which stores in advance predetermined data including the outside diameters of predetermined syringes of a plurality of kinds, and at least the capacity of syringe and the travel distance of plunger, which correspond to the syringe of each outside diameter.

FIG. 1

FIG. 2 （a）

FIG. 2 （b）

# FIG. 3

RAM DISC ~14a

INITIAL SYRINGE DATA STORAGE SECTION 14b

DISTANCE CALCULATION PROGRAM ~17a
CAPACITY CALCULATION PROGRAM ~17b
DIFFERENCE CALCULATION PROGRAM ~17c
REGISTRATION PROGRAM ~17d
CONTROL PROGRAM ~17e

ROM

RAM ~19

INPUT SECTION 13

CPU 18

DISPLAY SECTION 12

10

11

15

9

4    4b

5

## FIG. 4

START

STEP1
SET MODE

STEP2
INPUT CAPACITY AND MAKER NAME

STEP3
PULL PLUNGER BEYOND MAXIMUM SCALE

STEP4
HOLD SYRINGE

STEP5
MEASURE OUTSIDE DIAMETER

STEP6
SET AT MAXIMUM SCALE POSITION ?
NO

YES STEP7
PRESS

STEP8
TO SYRINGE DISTAL END ?
NO

YES STEP9
STOP PRESSING

STEP10
CALCULATE DISTANCE

STEP11
CALCULATE CAPACITY

STEP12
DIFFERENCE IN CAPACITY WITHIN PREDETERMINED RANGE ?
NO

YES STEP13
STORE

END

FIG. 5

```
        ( START )
            |
            |          STEP21
  +------------------+
  |   HOLD  SYRINGE  |
  +------------------+
            |
            |          STEP22
  +------------------+
  | SELECT MAKER NAME|
  +------------------+
            |
            |          STEP23
  +------------------+
  |  CHECK  SYRINGE  |
  +------------------+
            |
            |          STEP24
  +------------------+
  |  SET  FLOW  RATE |
  +------------------+
            |
            |          STEP25
  +------------------+
  | CONNECT TO PATIENT|
  +------------------+
            |
            |          STEP26
  +------------------+
  |  START INJECTION |
  +------------------+
            |
            |
         ( END )
```

# EP 1 561 949 A1

## INTERNATIONAL SEARCH REPORT

International application No.

PCT/JP03/14110

### A. CLASSIFICATION OF SUBJECT MATTER
Int.Cl⁷ F04B49/06, A61M5/14

According to International Patent Classification (IPC) or to both national classification and IPC

### B. FIELDS SEARCHED

Minimum documentation searched (classification system followed by classification symbols)
Int.Cl⁷ F04B49/06, A61M5/14

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2003 |
| Kokai Jitsuyo Shinan Koho | 1971–2003 | Toroku Jitsuyo Shinan Koho | 1994–2003 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | WO 88/10383 A1 (THE UNIVERSITY OF MELBOURNE), 29 December, 1988 (29.12.88), & JP 2625533 B2 & US 5034004 A1 & EP 434672 A | 1-3 |
| Y | JP 2000-70365 A (Japan Servo Co., Ltd.), 07 March, 2000 (07.03.00), (Family: none) | 1-3 |
| Y | JP 63-39285 B2 (Terumo Corp.), 04 August, 1988 (04.08.88), (Family: none) | 1-3 |
| Y | JP 5-58347 B2 (Nikkiso Co., Ltd.), 26 August, 1993 (26.08.93), (Family: none) | 1-3 |

☐ Further documents are listed in the continuation of Box C.  ☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 15 December, 2003 (15.12.03) | 13 January, 2004 (13.01.04) |

| Name and mailing address of the ISA/ | Authorized officer |
|---|---|
| Japanese Patent Office | |
| Facsimile No. | Telephone No. |

Form PCT/ISA/210 (second sheet) (July 1998)